# EUROPEAN PATENT APPLICATION

(11) **EP 2 135 604 A1**
(43) Date of publication of application: **23.12.2009**
(21) Application number: 08738888.0
(22) Date of filing: 26.03.2008
(51) Int. Cl.: A61K 31/198, A61K 9/08, A61P 3/02, A61P 35/00

(54) **INFUSION PREPARATION FOR CANCER PATIENT**

(30) Priority: 26.03.2007 JP 2007080011
(71) Applicant: Matsui, Hirofumi, Tsukuba-shi Ibaraki 305-0031 (JP)
(72) Inventor: Matsui, Hirofumi, Tsukuba-shi Ibaraki 305-0031 (JP)
(74) Representative: Nicholls, Kathryn Margaret
(86) International application number: PCT/JP2008/055700
(87) International publication number: WO 2008/123298

(57) **Abstract**

Provided is a side-effect-free amino acid infusion preparation for use with terminal cancer patient that can effectively suppress the growth/proliferation of tumors in patients with advanced or terminal cancer while effectively ameliorating malnutrition in such patients. The high-amino acid infusion preparation can be used for the nutritional management in patients with advanced or terminal cancer. Specifically, the high-amino acid infusion for nutritional management of patients with advanced or terminal cancer is a low-sugar, high-amino acid infusion containing high levels of L-glutamine or glycine. Also provided is a method for treating cancer, including administering a high-amino acid infusion for a long-term period for nutritional management of patients with advanced or terminal cancer.

## Description

### TECHNICAL FIELD

The present invention relates to an infusion containing high levels of amino acids that is intended to be used for the nutritional management in cancer patients, in particular, terminal cancer patients to ameliorate malnutrition in the patients and thus treat cancer of the patients.
In particular, the present invention relates to a high-amino acid infusion preparation that contains high levels of amino acids to be administered in varying composition so as to ameliorate malnutrition in patients with advanced and/or terminal cancer and thereby suppress the growth/proliferation of tumors in the cancer patients.

### BACKGROUND ART

What is known as amino acid imbalance therapy has recently been used to suppress the growth/proliferation of tumors. Specifically, the therapy inhibits the synthesis of nucleic acids and, thus, the synthesis of corresponding proteins in tumor cells by inducing amino acid imbalance. This can be done by varying the composition of amino acids administered, or depleting or increasing particular amino acids.
Different preparations for use in the amino acid imbalance therapy have been proposed thus far and are considered effective in their own way.

For example, Patent Document 1 proposes an amino acid preparation for use in cancer patients that contains relatively high levels of L-arginine and branched-chain amino acids L-leucine, L-isoleucine and L-valine and relatively low levels of L-aspartic acid and L-glutamic acid. This preparation contains relatively high levels of essential amino acids other than L-arginine.
Patent Document 2 proposes an amino acid preparation for use in cancer patients that contains relatively high levels of L-arginine and relatively low levels of L-methionine and contains no non-essential amino acids including L-glutamic acid and L-aspartic acid.
Each of these amino acid preparations for cancer patients is characteristic in its amino acid imbalance in which the levels of particular amino acids are varied. While they can improve the nutritional condition in cancer patients, their ability to suppress tumor growth is insufficient.

To overcome this drawback, an amino acid infusion preparation for use in cancer patients has been proposed that has the ability to suppress the tumor growth in conjunction with the ability to improve the nutritional condition of cancer patients (Patent Document 3). Though effective to some extent, this preparation is still less than satisfactory to improve the nutritional condition and suppress the growth/proliferation of tumors particularly in patients with advanced or terminal cancers.
Thus, there is a need for the development of amino acid infusion preparations that can ameliorate malnutrition in patients with advanced or terminal cancer and can suppress the growth/proliferation of tumors, so that they can be used to effectively treat, or prolong the life of, patients with advanced or terminal cancer.

Patent Document 1 Japanese Patent Publication No. Hei 5-79049
Patent Document 2 Japanese Patent No. 2537406
Patent Document 3 Japanese Patent No. 3429327
Patent Document 4 International Patent Publication No. WO 2006/043336

### DISCLOSURE OF THE INVENTION

### PROBLEMS TO BE SOLVED BY THE INVENTION

In view of the current situation described above, it is an object of the present invention to provide a side-effect-free amino acid infusion preparation for use in terminal cancer patients, the preparation that can effectively suppress the growth/proliferation of tumors in patients with advanced or terminal cancer while effectively ameliorating malnutrition in terminal cancer patients.

### MEANS FOR SOLVING THE PROBLEMS

In the course of studies to find ways to achieve the foregoing object, the present inventor has found that by replacing the intravenous hyperalimentation (IVH), a current nutritional therapy offered to terminal cancer patients, with peripheral parenteral nutrition (PPN) to prevent infections caused by catheters, a major problem associated with IVH, the life expectancy of terminal cancer patients can be significantly prolonged. The reason for this is considered to be that only limited amounts of glucose, a major energy source for tumor cells, are available.

The present inventor has also found that decreasing the glucose levels in the culture medium results in decreased protein levels in tumor cell lines of rat and human stomach cancer in a concentration-dependent manner, as opposed to the normal stomach mucous cell line of rat, in which the decreased glucose levels cause no changes in the protein levels. This indicates that reducing glucose levels can decrease the activity of the tumor cells.
The present inventor has further found that energy production via oxidative phosphorylation in mitochondria is impaired in tumor cells. The inventor thus hypothesized that supplying sufficient amounts of particular amino acids that are utilized by metabolic enzymes in the TCA cycle in mitochondria for energy production can provide an energy source that can be utilized by normal cells, but not by tumor cells, thus leading to suppressed growth/proliferation of tumor cells.
These findings have led the present inventor to speculate that an infusion containing low sugar and high amino acids in particular composition can be administered to patients with advanced or terminal cancer to maintain and ameliorate nutritional conditions of the cancer patients while suppressing the growth/proliferation of tumors in these patients.

Accordingly, one essential aspect of the present invention concerns a high-amino acid infusion for nutritional management of patients with advanced or terminal cancer.

More specifically, the high-amino acid infusion of the present invention for nutritional management of patients with advanced or terminal cancer is a low-sugar, high-amino acid infusion containing a high level of L-glutamine or glycine.

More preferably, the high-amino acid infusion of the present invention for nutritional management of patients with terminal cancer is a low-sugar, high-amino acid infusion containing no sugar.

Specifically, the present invention concerns the high-amino acid infusion in which the nutritional management of patients with advanced or terminal cancer involves ameliorating malnutrition of the cancer patients and suppressing the growth/proliferation of tumors. The present invention further concerns a therapeutic composition for cancer including the high-amino acid infusion.

Another aspect of the present invention concerns a method for treating cancer, including administering a high-amino acid infusion for a long-term period for nutritional management of patients with advanced or terminal cancer.

More specifically, the method for treating cancer includes performing nutritional management of patients with advanced or terminal cancer by using the high-amino acid infusion being a low-sugar, high-amino acid infusion, in particular, containing high levels of L-glutamine or glycine. More preferably, the method for treating terminal cancer includes using the high-amino acid infusion being a low-sugar, high-amino acid infusion containing no sugar in nutritional management of patients with advanced or terminal cancer.

The method for treating cancer according to this aspect of the present invention involves ameliorating malnutrition of the patients with advanced or terminal cancer and suppressing the growth/proliferation of tumors through administration of the low-sugar, high-amino acid infusion.

### EFFECTS OF THE INVENTION

The high-amino acid infusion or the low-sugar, high-amino acid infusion provided in accordance with the present invention can be administered to patients with advanced and/or terminal cancer to ameliorate malnutrition of the patients and suppress the growth/proliferation of tumors, thus prolonging the life of terminal cancer patients.
This compares with any of the previously proposed amino acid preparations for use in cancer patients, which can suppress the growth/proliferation of tumors, but cannot improve nutritional conditions of cancer patients. In addition, each of the previously proposed amino acid infusion preparations for use in cancer patients is not intended for use in terminal cancer patients and has therefore limited ability to prolong the life of patients with advanced or terminal cancer.
The high-amino acid infusion of the present invention for nutritional management of patients with advanced or terminal cancer is unique in that it can ameliorate malnutrition in patients with advanced or terminal cancer and suppress the growth/proliferation of tumors, thereby prolonging the life of patients with advanced or terminal cancer patients.

### BRIEF DESCRIPTION OF THE DRAWINGS

Fig. 1 is a diagram showing the BrdU uptake by a rat stomach mucous membrane cell line obtained from a rat with a growing tumor (non-tumor cells) and a rat stomach cancer cell line (tumor cells) after cultured for 24 hours in a growth medium containing different concentrations of glucose, according to Example 1 of the present invention.
   The BrdU uptake for each culture is shown relative to the uptake obtained at a glucose concentration of 3g/L (= 1) in the growth medium.
Fig. 2 is a diagram showing the ability to consume oxygen for a rat stomach mucous membrane cell line (non-tumor cells) and different rat stomach cancer cell lines (tumor cells), according to Example 2 of the present invention.
   The oxygen consumption for each cell culture is shown relative to the oxygen consumption rate of the rat stomach mucous membrane cell line (= 1).
Fig. 3 is a diagram showing the mitochondria activity of a rat stomach mucous membrane cell line (non-tumor cells) and different rat stomach cancer cell lines (tumor cells) after cultured for 12 to 24 hours in a growth medium containing Rotenone (electron transport chain I inhibitor) or Antimytin A, each an inhibitor of the electron transport chain in mitochondria, according to Example 3 of the present invention.
   The mitochondria activity of each cell culture is measured as the survival rate using TC-1 (Toxicolor) and is shown relative to the survival rate measured before the addition of each inhibitor (= 100).

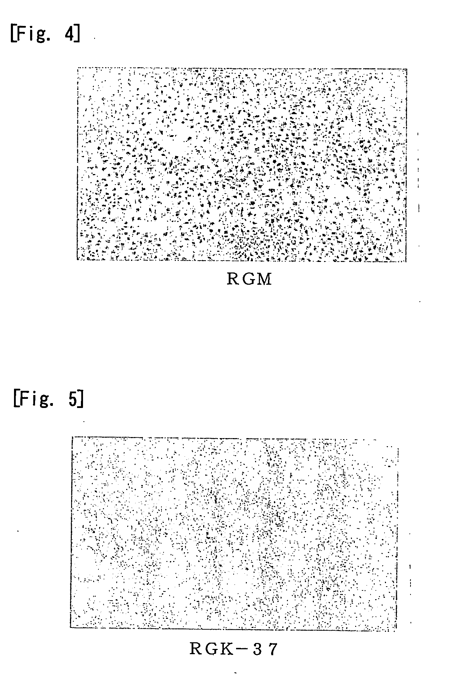

Fig. 4 is a micrograph of a rat stomach mucous membrane cell line obtained from a rat with a growing tumor (RGM/non-tumor cells) after cultured for 120 hours in a glucose-free growth medium containing 3% glutamine.
Fig. 5 is a micrograph of a rat stomach cancer cell line obtained from a rat with a growing tumor (RGK-37/ tumor cells) after cultured for 120 hours in a glucose-free growth medium containing 3% glutamine.

### DESCRIPTION OF REFERENCE NUMERALS

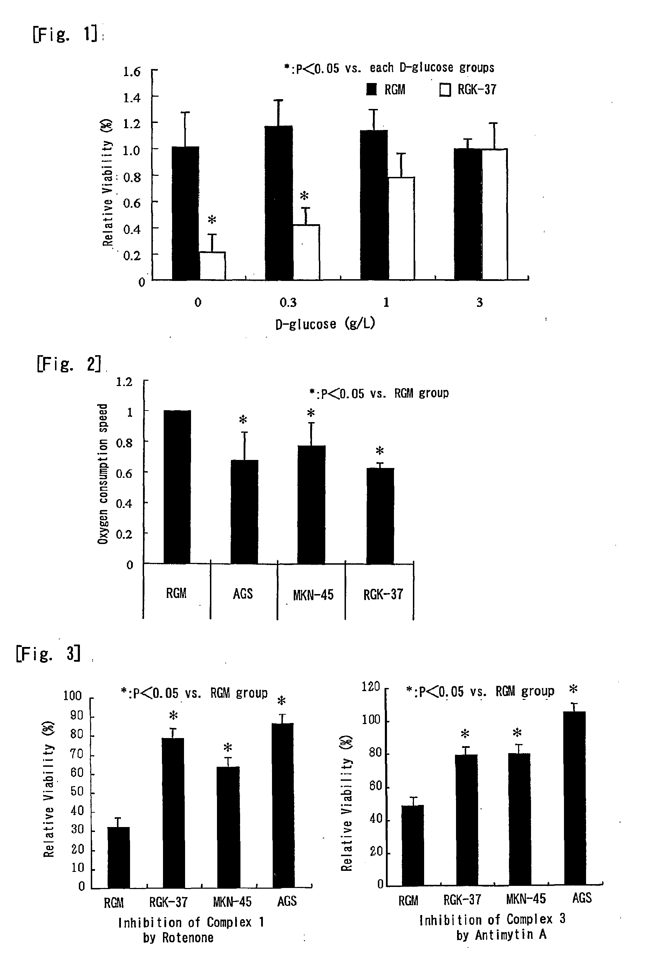

The statistical test used in Figs. 1 to 3 was Dunnett's test with a significant level of 5%. A p-value less than 5% was considered to be a statistically significant difference.
Data for each group was given in average ± standard deviation.

### BEST MODE FOR CARRYING OUT THE INVENTION

The high-amino acid infusion of the present invention for use in patients with terminal cancer is intended for use in inoperable patients with advanced or terminal cancer who cannot or have difficulty ingesting nutritional sources orally and are under malnutrition, so as to provide a protein source needed to improve hypoproteinemea/hypoalbuminemea and N-balance. Thus, the high-amino acid infusion of the present invention can effectively deliver nutrients to the patients in need thereof. The high-amino acid infusion of the present invention has a feature in that it employs as a calorie source amino acids, which can be readily utilized by normal cells, rather than sugar.

A study conducted by the present inventor has revealed that tumor cells have unique energy production characteristics that distinguish them from normal cells. Patients with terminal cancer often experience dyscrasia. Once this happens, it is difficult to improve the deteriorated nutritional conditions even with a supply of nutrients, leading to malnutrition. Many of terminal cancer patients develop insulin resistance that suppresses the sugar uptake by the body. Thus, sugar cannot be utilized as an energy source in such patients, who will remain malnourished even if calorie sources are administered.
In contrast, it has been reported that many tumor cells have increased mRNA expression of GLUT1, a glucose transporter. Analysis with positron emission tomography (PET) imaging also shows increased sugar uptake by the tumor cells. Tumor cells are also reported to show increased activity of lactose dehydrogenase (LDH). These observations suggest that anaerobic sugar metabolism in the glycolysis is highly increased in tumor cells. It is known that lactic acid produced in the glycolysis uses a process known as "co-recycle," in which it is converted back into glucose primarily in the liver and becomes available to tumor cells again.

Using a fluorescence endoscope, the present inventor has often observed that human stomach cancer tissue emits fluorescence. This is believed to be due to the increased porphyrin uptake by the tumor cells. Porphyrin incorporates Fe²⁺ by the action of ferrochelatase present on the mitochondrial membrane and is converted into heme, which in turn enters the electron transport chain present on the inner mitochondrial membrane. It is speculated that high expression levels of NO synthase in tumor cells cause oxidative damage to ferrochelatase by NO radicals produced in the cytoplasm of the tumor cells. As a result, the enzymatic activity of ferrochelatase is inhibited, resulting in the accumulation of porphyrin in the cells.
The present inventor has considered that since the supply of cytochromes comes short in tumor cells, proton transfer in the electron transport chain is disrupted, so that the ATP production by aerobic oxidation is inhibited. As a result, tumor cells are dependent on specific metabolism that relies on the glycolysis to produce energy.

Since tumor cells have disrupted electron transport chain that cannot produce ATP effectively, limiting the sugar supply to tumor cells can further suppress the production of ATP by tumor cells and thus prevent the tumor growth. Instead, most of the daily calories may be delivered in the form of amino acids or peptides to allow the host cells to utilize the amino acids or peptides in the citric acid cycle (i.e., TCA cycle) to produce ATP. Establishing the host environment in which the tumor growth is suppressed may also enhance the effects of chemotherapy.

The amino acid for use in the high-amino acid infusion of the present invention may be provided not only in its free form, but also in the form of pharmaceutically acceptable salts, such as metal salts formed with metals such as sodium and potassium, inorganic acid salts, such as hydrochlorides and sulfates, or organic acid salts, such as acetates and citrates.
Some or all of the amino acids may be provided in the form of N-acyl derivatives or N-acetyl derivatives.
Alternatively, the amino acids may be provided in the form of a peptide composed of two or more different amino acids bound to one another via peptide bonds.

The high-amino acid infusion of the present invention preferably contains particular types of amino acid that can be effectively utilized by non-tumor cells, but not by tumor cells. It turns out that the presence of high levels of L-glutamine is particularly important for such an amino acid composition. L-glutamine, also known as a conditional essential amino acid whose required amount increases upon exposure to invasive stress, is the amino acid that can be most effectively utilized in energy production. Patent Document 4 reports that L-glutamine has an ability to suppress the onset of legions resembling stomach cancer in rats infected with *Helicobacter pylori.* The present inventor has also found that addition of L-glutamine to the growth medium suppresses chemical carcinogenesis in rat stomach mucous membrane cells, and that non-tumor cells can survive in a sugar-free/high L-glutamine medium, whereas apoptosis is induced in tumor cells in such a growth medium. These observations suggest that L-glutamine is the most suitable amino acid for use in cancer patients in terms of both energy production and suppression of carcinogenesis.

Glycine, an amino acid metabolized by the glycine cleavage enzyme present on the inner mitochondrial membrane to be utilized in the ATP production, is less effectively utilized by certain types of cancer in which mitochondrial functions are impaired.
Accordingly, it is important that amino acids, mainly essential amino acids, be present in the high-amino acid infusion of the present invention in a well-balanced manner in order to maintain and improve the nutritional conditions of patients. Preferably, the high-amino acid infusion of the present invention contains high proportions of L-glutamine and glycine while maintaining nutritional balance.

Preferred specific ranges for the amounts of amino acids in the amino acid composition are shown in Table 1 below.

**(Table 1)**

| Amino acids | Ranges of amounts (g/100g total amino acids) |
|---|---|
| L-isoleucine | 0.1 - 5.0 |
| L-leucine | 0.2 - 9.0 |
| L-valine | 0.2 - 10.0 |
| L-lysine | 0.15 - 6.0 |
| L-methionine | 0.1 - 5.0 |
| L-phenylalanine | 0.1 - 7.7 |
| L-threonine | 0.1 - 5.0 |
| L-tryptophan | 0.02 - 3.0 |
| L-alanine | 0.1 - 20.0 |
| L-arginine | 0.1 - 11.3 |
| L-aspartic acid | 0.02 - 5.0 |
| L-cysteine | 0.01 - 0.7 |
| L-glutamic acid | 0.01 - 3.6 |
| L-glutamine | 0.1 - 30.0 |
| L-histidine | 0.1 - 4.1 |
| L-proline | 0.1 - 5.9 |
| L-serine | 0.05 - 2.0 |
| L-tyrosine | 0.0 - 1.0 |
| Glycine | 0.1 - 5.4 |

The high-amino acid infusion of the present invention for nutritional management of patients with advanced or terminal cancer contains high levels of particular amino acids, along with electrolytes added in a well-balanced manner and, if necessary, a small amount of glucose. A preferred composition is as follows:

| | |
|---|---|
| L-isoleucine | 0.8 - 5.0g /L |
| L-leucine | 1.0 - 9.0 g/L |
| L-valine | 1.0 - 10.0 g/L |
| L-lysine | 0.8 - 6.0 g/L |
| L-methionine | 0.1 - 3.0 g/L |
| L-phenylalanine | 0.5 - 5.0 g/L |
| L-threonine | 0.1 - 5.0 g/L |
| L-tryptophan | 0.05 - 1.0 g/L |
| L-alanine | 1.0 - 20.0 g/L |
| L-arginine | 0.01 - 10.0 g/L |
| L-aspartic acid | 0.08 - 2.0 g/L |
| L-cysteine | 0.02 - 1.2 g/L |
| L-glutamic acid | 0.04 - 2.0 g/L |
| L-glutamine | 2.0 - 30.0 g/L |
| L-histidine | 0.3 - 3.0 g/L |
| L-proline | 0.4 - 5.0 g/L |
| L-serine | 0.1 - 1.0 g/L |
| L-tyrosine | 0.0 - 0.5 g/L |
| glycine | 1.0 - 20.0 g/L |
| Na⁺ | 20 - 150 mEq/L |
| K⁺ | 2 - 50 mEq/L |
| Ca²⁺ | 0 - 20 mEq/L |
| Mg²⁺ | 0 - 20 mEq/L |
| Cl⁻ | 20 - 150 mEq/L |
| P | 0 - 20 mmol/L |
| Zn²⁺ | 0 - 50 mmol/L |
| glucose | 0 - 50 g/L |

When the high-amino acid infusion of the present invention is administered peripherally (PPN), the osmotic pressure of the infusion is preferably adjusted to 3 or less to avoid the risk of vascular pain and venous inflammation. Preferred specific ranges for the amounts of the components are as follows:

| | |
|---|---|
| Amino acids in the composition of Table 1 | 40 - 60 g/L |
| Na⁺ | 20 - 60 mEq/L |
| K⁺ | 2 - 30 mEq/L |
| Ca²⁺ | 0 - 10 mEq/L |
| Mg²⁺ | 0 - 10 mEq/L |
| Cl⁻ | 20 - 4 mEq/L |
| P | 0 - 20 mmol/L |
| Zn²⁺ | 0 - 50 µmol/L |
| glucose | 0 - 50 g/L |

The individual components of the infusion of the present invention may be separately contained in multiple compartments defined in a container that are separated by a separator that can be ruptured to allow the compartments to communicate to form a single chamber for mixing of the components. In one case, for example, a first chamber is filled with a sugar solution while a second chamber is filled with an amino acid solution. In another case, some of the electrolytes are separately contained in a compartment other than the first chamber and the second chamber. In still another case, vitamins are separately contained in a compartment other than the first chamber and the second chamber.

The high-amino acid infusion of the present invention for nutritional management of patients with advanced or terminal cancer is prepared by formulating the above-described amino acids or derivatives thereof or peptides in the form of free amino acids. Any preparation method used to prepare typical nutrient infusion preparations or amino acid infusion preparations may be used. In a typical method, for example, the above-described amino acids or derivatives thereof are dissolved in distilled water for injection to form an aqueous solution. If necessary, a stabilizer, such as sodium bisulfate, a pH conditioner (for example, hydrochloric acid, acetic acid, lactic acid, malic acid, citric acid or sodium hydroxide) and other additives are added. The resulting aqueous solution is sterilized by heating or subjected to sterile filtration to form the desired infusion preparation.

The thus prepared high-amino acid infusion preparation typically has a pH of 4.0 to 7.0, and preferably 5.5 to 7.0, and typically contains amino acids at a high concentration of 3 to 10wt%, and preferably 5 to 10wt%.

Unlike any of the previously proposed amino acid infusions, the high-amino acid infusion for nutritional management of patients with advanced or terminal cancer has a feature in that it contains minimal levels of glucose and other sugars that can be utilized as an energy source, but instead contains amino acids as the energy source. It is important that the infusion preparation contains low levels of glucose and other sugars or do not contain glucose and other sugars at all.
When the infusion preparation contains glucose as a sugar, it is important that its amount should be kept at 5wt% or less to ensure fluid replacement.

Optional components commonly used in amino acid infusions, including vitamins, electrolytes and trace elements, may also be added as desired.

The high-amino acid infusion of the present invention for nutritional management of patients with advanced or terminal cancer is administered intravenously through, for example, peripheral venous or central vein. The infusion may be administered at a dose commonly used for a typical amino acid infusion preparation: in general, it is administered at a dose of 200 to 2000mL per day per adult. Based on this dose range, the infusion may be administered in varying doses determined depending on the disease conditions, nutritional conditions, age and body weight of the terminal cancer patient to which the infusion is administered.

### [EXAMLES]

The present invention will now be described with reference to examples, which are not intended to limit the invention.

### Example 1: Effect of glucose concentration in the growth media on the growth of tumor cells

A rat stomach mucous membrane cell line (RGM) and a rat stomach cancer cell line (RGK-37) were cultured in Gibco#11330-032 DMEM/F12 medium and L-glutamine-free Sigma#D6421 DMEM/F12 medium, respectively. Each culture was supplemented with 10% FBS and was cultured during the logarithmic growth phase. The growth medium was then replaced with a growth medium containing 0, 0.3 or 1.3 g/L of glucose and supplemented with 1%FBS and 1% L-glutamine. After 24 hours of culturing, bromodeoxyuridine (BrdU) was added to each culture. 2 hours after the addition of BrdU, the BrdU uptake was measured by ELISA. The BrdU uptake for each culture is shown in Fig. 1 relative to the uptake obtained at a glucose concentration of 3 g/L (= 1).

The results indicate that the BrdU uptake was relatively constant in the non-tumor cells (RGM) independently of the glucose concentration, indicating substantially no change in the cell growth, whereas the BrdU uptake decreased relatively with decreasing concentrations of glucose in the stomach cancer cells (RGK-37), indicating suppression of cell growth.
Thus, it has been demonstrated that non-tumor cells can utilize amino acids, rather than glucose, in the growth medium as an energy source, whereas stomach cancer cells have a decreased ability to utilize amino acids, so that their growth ability decreases by limiting the sugar supply.

### Example 2: The ability of non-tumor and different stomach cancer cells to consume oxygen

A rat stomach mucous membrane cell line (RGM), a rat stomach cancer cell line (RGK-37), a human stomach cancer cell line (AGS) and another human stomach cancer cell line (MKN45) were cultured in Gibco#11330-032 DMEM/F12 medium, L-glutamine-free Sigma#D6421 DMEM/F12 medium, Sigma#N6658 F12 medium and Gibco#11875-093 RPM1640 medium, respectively. Each culture was supplemented with 10% FBS and was cultured during the logarithmic growth phase. The cells were treated with trypsin and 10⁷ cells were suspended in phosphate-buffered saline. Using an oxygen electrode (YSI5300A), the oxygen consumption after 5 minutes to after 10 minutes was measured to calculate the oxygen consumption rate. The calculated oxygen consumption rate for each cell culture is shown in Fig. 2 relative to the oxygen consumption rate of RGM (= 1).
The results suggest that each stomach cancer cell line has a decreased ability to consume oxygen when compared with RGM or a non-tumor cell and is therefore less dependent on the aerobic energy production.

### Example 3: Effect of electron transport chain inhibitors on the mitochondria activity in non-tumor and different stomach cancer cells

A rat stomach mucous membrane cell line (RGM), a rat stomach cancer cell line (RGK-37), a human stomach cancer cell line (AGS) and another human stomach cancer cell line (MKN45) were cultured in Gibco#11330-032 DMEM/F12 medium, L-glutamine-free Sigma#D6421 DMEM/F12 medium, Sigma#N6658 F12 medium and Gibco#11875-093 RPM1640 medium, respectively. Each culture was supplemented with 10% FBS and was cultured during the logarithmic growth phase. Rotenone (electron transport chain I inhibitor) or Antimytin A (electron transport chain III inhibitor), each an inhibitor of the electron transport chain in mitochondria, was added to each medium to a concentration of 100 µM or 1 µM, respectively. After 12 to 24 hours, the mitochondria activity of each cell culture was measured as the survival rate using TC-1 (Toxicolor). The survival rate of each culture was shown in Fig. 3 relative to the survival rate measured before the addition of each inhibitor (= 100).

The results indicate that the survival rate of non-tumor cells (RGM) was significantly decreased by the addition of each inhibitor, whereas no significant change was observed in the survival rate of each of the stomach cancer cell line.
This suggests that ATP is effectively produced by oxidative phosphorylation in the electron transport chain in non-tumor cells, whereas stomach cancer cells are less dependent on the aerobic energy production via the electron transport chain, so that they obtain energy by anaerobic respiration. In other words, it has been shown that stomach cancer cells obtain energy mainly from the glycolysis.

### Example 4:

A rat stomach mucous membrane cell line (RGM) and a rat stomach cancer cell line (RGK-37) were cultured in Gibco#11330-032 DMEM/F12 medium and Sigma#D6421 DMEM/F12 medium, respectively. Each culture was cultured in the absence of glucose and in the presence of 3% glutamine for 120 hours. Subsequently, the cells were observed under a microscope. The non-tumor cells (RGM) survived, whereas apoptosis was induced in the RGK-37 cell line. This observation suggests that the survival rate of non-tumor cells is not significantly affected under the sugar-free, high L-glutamine culture condition, whereas tumor cells cannot survive under the same condition.

### INDUSTRIAL APPLICABILITY

The high-amino acid infusion of the present invention, specifically a low-sugar/high-amino acid infusion containing high levels of L-glutamine and glycine as amino acids, can be administered to patients with advanced or terminal cancer to suppress the growth/proliferation of tumors and ameliorate malnutrition in such patients, thus prolonging the life of terminal cancer patients.
The high-amino acid infusion of the present invention also improves the quality of life of patients by ameliorating malnutrition of patients. The cytotoxins administered in conventional chemotherapies with the intension of inducing cytotoxic effects in a cancer-specific manner have strong side effects, the problem that needs to be addressed urgently. In particular, gastrointestinal side effects can deteriorate nutritional conditions of cancer patients and make it difficult to continue the chemotherapy.
Not only can the low-sugar/high-amino acid infusion of the present invention improve the nutritional condition of cancer patients, but it can also be used as an infusion that can exploit or maximize the effect of chemotherapy.

## Claims

1. A high-amino acid infusion for use in nutritional management of patients with advanced and/or terminal cancer.

2. The high-amino acid infusion according to claim 1, being a low-sugar, high-amino acid infusion.

3. The high-amino acid infusion according to claim 1 or 2, containing a high level of L-glutamine or glycine.

4. The high-amino acid infusion according to claim 1, 2 or 3, wherein the low-sugar, high-amino acid infusion contains no sugar.

5. The high-amino acid infusion according to claim 1, 2, 3 or 4, wherein the nutritional management of patients with advanced and/or terminal cancer involves ameliorating malnutrition of the cancer patients and suppressing the growth/proliferation of tumors.

6. A therapeutic composition for cancer, comprising the high-amino acid infusion according to any one of claims 1 to 5.

7. A method for treating cancer, comprising administering a high-amino acid infusion for a long-term period for nutritional management of patients with advanced and/or terminal cancer.

8. The method for treating cancer according to claim 7, wherein the high-amino acid infusion is a low-sugar, high-amino acid infusion.

9. The method for treating cancer according to claim 7 or 8, wherein the high-amino acid infusion contains a high level of L-glutamine or glycine.

10. The method for treating cancer according to claim 8, wherein the low-sugar, high-amino acid infusion contains no sugar.

11. The method for treating cancer according to claim 7, wherein the nutritional management of patients with advanced and/or terminal cancer involves ameliorating malnutrition of the cancer patients and suppressing the growth/proliferation of tumors.
